# EUROPEAN PATENT SPECIFICATION

(11) **EP 0 436 598 B1**
(45) Date of publication and mention of the grant of the patent: **13.03.1996**
(21) Application number: 89910722.1
(22) Date of filing: 15.09.1989
(51) Int. Cl.: G01N 33/569, C12N 5/00, C07K 16/28, C12P 21/00

(54) **CELL SORTING TECHNIQUE AND APPLICATIONS THEREOF**
ZELLENAUSSORTIERUNGSTECHNIK UND IHRE ANWENDUNGEN
TECHNIQUE DE TRIAGE DE CELLULES ET SES APPLICATIONS

(30) Priority: 27.09.1988 US 249710
(43) Date of publication of application: 17.07.1991
(73) Proprietor: CETUS ONCOLOGY CORPORATION, Emeryville California 94608 (US)
(72) Inventor: Shi, Tim, Pinole, CA 94564 (US)
(74) Representative: Bizley, Richard Edward
(86) International application number: US8904023
(87) International publication number: WO9003576

(56) References cited:
- EP-A- 0 105 614
- EP-A- 0 241 042
- US-A- 4 499 052
- JOURNAL OF IMMUNOLOGICAL METHODS, vol. 96, 1987, Elsevier Science Publishers B. V. (Biomedical Division); L. KARAWAJEW et al., pp. 265-270/
- CHEMICAL ABSTRACTS, vol. 108, no. 19, 09 May 1988, Columbus, OH (US); p. 522, no. 166012d/
- CHEMICAL ABSTRACTS, vol. 108, no. 3, 18 January 1988, Columbus, OH (US); A. KRISHAN, p. 300, no. 18663b/
- Proc.Natl.Acad.Sci.USA(1982) 79 3275-3279

## Description

This invention is in the field of cell biology, and is concerned with a cell sorting technique whereby certain hybrid cells are identified and isolated. More specifically, certain hybrid antibody secreting cells may be selected using a fluorescence activated cell sorter.

Techniques which permit the formation of hybrid cells have significantly facilitated an understanding of various biological phenomena, as well as having had profound practical applications, most notably the formation of antibody secreting hybrid cell-lines. Kohler, G. et al Nature 256:495 (1975). Basically, what is involved in forming hybrid cells is fusing one or more target cells with an appropriate fusogen, and subsequently selecting out of the mixture the hybrid cells. Methods for fusing cells, as well as methods for selecting hybrids are known in the art.

The two most widely employed fusogens, or cell fusing chemicals, are polyethylene glycol (PEG) or inactivated virus, particularly Sendai virus. Ringertz, N. and Salvage, R. Cell Hybrids Chapters 4 (p. 29) and 5 (p. 46), Academic Press (1976). Because of the ready availability of PEG, and because Sendai virus requires time-consuming production, titration and inactivation steps, PEG is by far the favored fusogen. PEG is available in various molecular weight formulations, and particular formulations are preferred fusogns. Gefter, M. et al., Somat Cell Genet. 2:231, (1977). Hybrid cells are generally selected using one of two methods. The first consists of forming hybrids in defined media, but not the non-fused cells. The second consists of introducing two distinct fluorescent markers into each of the cells that make up the hybrids, and subsequently, selecting hybrids that exhibit both fluorescent molecules using a fluorescent activated cell sorter machine.

Thus, reference can be made to J. Immun. Mtds (1987) 96 265-270 and DD 0 249 492. In Cytometry (1987) 8(6) 642-645, a calcium blocker such as verapamil is used in regard to overcoming the problem of dye retention in cells in fluorometric assays.

Fluorescent activated cell-sorting techniques have several advantages over isolating hybrids using classical biochemical selection techniques. First, and most apparent is that there is no need to produce cell lines having the genetic markers that permit selection in drug supplemented media. This is often a very time consuming and arduous task. Second, after the fusion procedure is carried out, a biochemical selection procedure is not required wherein the hybrids and non-fused cells are grown in drug selection medium in order to select against the non-fused cells. Third, the efficiency of a particular fusion technique can be determined immediately using fluorescent activated cell sorter techniques. In contrast, if the cells are selected using drug markers it is necessary to wait a significant period of time for the cultures to grow up, and be depleted of non-fused cells.

Despite the advantages of selecting hybrid cells using fluorescent activated cell sorting techniques, the method is not generally applicable to a wide variety of cells because of the difficulties associated with identifying compatible combinations of dyes. This is particularly true if mouse hybrids are selected because of the paucity of fluorescent dyes that are taken up by the cells, and additionally retained therein for a time sufficient to carry out the selection process. This is particularly true of mouse antibody secreting cells, either hybridomas, triomas, or quadromas. In part, this is because mouse cells are known to have membrane transport systems that can remove from the cell fluorescent dyes that are useful in fluorescent activated cell sorting.

Another drawback associated with fluorescent activated cell sorting techniques is that, to date, there have not been identified pairs of fluorescent dyes that can distinguish between dead and viable cells.

The present invention provides a method of producing a hybridoma capable of secreting antibody having bifunctional cellular cytotoxicity and recognising human Fc receptor III and the antigen recognised by the antibody produced by the 520C9 hybridoma comprising:
(a) contacting one of 3G8 cells, as described by Fleit et al, Proc. Natl. Acad. Sci. USA, 1982, 79: 3275-3279, and 520C9 cells, as described in US Patent No 4753894, with a physiological solution containing a first fluorescent dye for a time and at an effective concentration that permits said cells to become labelled with an amount of said first fluorescent dye detectable by a fluorescent activated cell sorting machine, said first fluorescent dye having a negligible rate of efflux from said cells;
(b) contacting the other of said 3G8 and 520C9 cells with a physiological solution containing a second fluorescent dye for a time and at an effective concentration that permits said other cells to take up an amount of said second fluorescent dye detectable by a fluorescent activated cell sorting machine and an effective amount of a calcium channel blocker that maintains said fluorescent dye at a detectable level inside said other cells;
(c) isolating said 3G8 and 520C9 cells from the respective physiological solutions;
(d) combining in a physiological solution the resulting 3G8 and 520C9 cells in the presence of a fusogen to produce a mixture containing hybrid and non-hybrid cells; and
(e) isolating hybrid cells from said mixture comprising passing said mixture through a fluorescent activated cell sorting machine.

The invention also includes a hybridoma capable of secreting antibody having bifunctional cellular cytotoxicity recognising human Fc receptor III and breast cancer cell antigen 520C9 and obtainable by a method of any one of claims 1 to 5. The invention further includes antibody produced by such a hybridoma and having bifunctional cellular cytotoxicity and recognising human Fc receptor III and breast cancer cell antigen 520C9.

Figure 1 shows the growth curve of 520C9 cells labeled with either rhodamine 123 at 0.5ug/ml, or hydroethidine at 10ug/ml. Also shown is the growth curve of control cells not labeled with either dye.

Figure 2 shows the growth curve of 3G8 cells labeled with rhodamine 123 at 0.5ug/ml, or hydroethidine at 5ug/ml or 10ug/ml. Also shown is the growth curve of control cells not labeled with either dye.

Figure 3 shows the FACS profile of 520C9 cells labeled with rhodamine 123.

Figure 4 shows the FACS profile of 3G8 cells labeled with hydroethidine.

Figure 5 shows the FACS profile of a mixture of unfused 520C9 and 3G8 cells.

Figure 6 shows the FACS profile of a mixture of cells containing fused 520C9 and 3G8 cells.

Figure 7 presents the chromomycin A3 DNA staining profile of 520C9 and 3G8 cells, and 3E11, a hybrid hybridoma derived from the fusion of these cell lines.

Figure 8 shows the chromomycin A3 DNA staining profile of 520C9 and 3G8 cells and 4H3, a hybrid hybridoma derived from the fusion of these cell lines.

Figure 9 shows the cytotoxic effect of 5A5, and 4H3 culture supernatants towards labeled SKBr3 cells.

Figure 10 shows the cytotoxic effects of 5A5, 4H3, and corresponding subclones towards tritium labeled SKBr3 cells.

Figure 11 illustrates the OD₂₈₀ Sephacryl (Trade Mark) 200 elution profile of ascites fluid containing antibody secreted by the hybrid hybridoma, 2B1.

Figure 12 shows the OD₂₈₀ DEAE Sepharose (Trade Mark) chromatography elution profile of antibody fractions of 2B1.

Figure 13 shows the SDS PAGE gel electrophoretic pattern of the DEAE fractions 52-62, 78-85 and 102-110.

Figure 14 shows the cytotoxic activity of DEAE purified 2B1 against chromium labeled SKBr3 cells.

The invention shown herein describes a method for selecting hybrid cells using fluorescent activated cell sorting techniques. Generally this involves selecting a pair of fluorescent dyes that are not toxic at the concentrations employed, and that are taken up intracellularly and retained therein for a time sufficient to perform the cell sorting procedure. Additionally, the fluorescent dyes will have the further property of exhibiting close excitation wavelengths but distinct emission wavelengths. The cells are fused using a fluorescent activated cell sorting machine.

### Labeling of Cells with Fluorescent Dyes

The invention is used to obtain cell lines that secrete bifunctional monoclonal antibodies. A bifunctional antibody exhibits two distinct antigen binding sites. Triomas and quadromas are two examples of cell lines that can secrete bifunctional monoclonal antibodies. Triomas are generally formed by fusion of a hybridoma and a lymphocyte, whereas quadromas are generally formed by somatic cell fusion of two hybridomas. The hybridomas and lymphocytes each produce a monospecific antibody, that is, an antibody that exhibits two binding sites for the same antigen. However, triomas and quadromas synthesize light and heavy chains of both parental types, that is, of both the hybridomas or lymphocytes, which combine to produce bispecific antibody.

The initial step in the identification and isolation of hybrid cells by the methods of the invention involve incubating, in a suitable physiological solution, those cells which are sought to be fused in separate tubes containing the fluorescent dyes of choice. The cells are allowed to incubate until an amount of fluorescent dye is taken up which is sufficient to be subsequently detected in a hybrid.

A wide variety of physiological solutions can be used to incubate cells in the presence of the fluorescent dyes. For instance, the cells can be incubated for short periods of time in physiological solutions lacking nutrients, such as phosphate buffered saline, or if a particular fluorescent dye requires longer incubation times to effect uptake of detectable amounts of dye, then the cell line is more appropriately incubated in a solution supplemented with nutrients, preferably cell culture media, to prevent cellular deterioration. The pH of the solution is expected to be in the range of about 7.4; however, variations are anticipated to be usable that do not significantly affect the uptake of the fluorescent dyes, nor adversely affect the viability of the cells.

The effect of fluorescent dyes on cell viability can be measured using techniques well known to those skilled in the art. A most useful technique is described by Mosmann, T., J. of Immunol. Methods, 65:55 (1983) which measures cell viability as a function of cell number. Thus, the effect of fluorescent dyes on both cell viability and cell number can be measured simultaneously. The assay is based on the conversion of colorless tetrazolium salts by mitochrondrial dehydrogenase enzymes to a detectable colored product. A favored tetrazolium salt is MTT, or (3-(4,5- dimethylthylthiazol-2-yl)-2,5- diphenyl tetrazolium bromide).

The fluorescent dyes that are usable in the instant invention have several important characteristics. First, as mentioned above, they should not be toxic to cells at the concentrations necessary to form and detect viable hybrids using a fluorescent activated cell sorter. Second, the dyes should also emit light at wavelengths that are readily detectable using suitable fluorescence detection devices. Third, once the dyes are taken up by the cells the rate of efflux of the dyes from the cells during the washing and measurement period should not be so great as to reduce the amount of dye inside the cell to a non-detectable level. Virtually any dye pair having these properties will perform adequately in the instant invention. However, most preferred are the dyes rhodamine 123 and hydroethidine. In most cell types these dyes are readily taken up and maintained. However, rodent cells, particularly mouse cells, exhibit a high efflux rate for rhodamine 123. It has been determined, however, that if cells that are incubated with rhodamine 123 are also incubated with a calcium channel blocker, that is, a molecule that interferes with the transport of calcium across the plasma membrane, that rhodamine 123 is maintained inside the cells for longer times than if the channel blocker is absent. Without wishing to be held to any particular theory, it is thought that rhodamine 123 is exported from the cell by a mechanism that is linked to calcium ion transport, and that by interfering with calcium ion transport using a suitable calcium channel blocker that efflux of rhodamine 123 is also blocked, and the dye is hence maintained inside the cell. Thus, in those instances where rhodamine 123 is one member of the dye pair, and the cells that are sought to be fused are rodent cells, in order to maintain a sufficient concentration of rhodamine 123 intracellularly, a calcium channel blocker should be present in the solution during the time that the cells are labeled with the dye, and during the subsequent washing, cell fusing, and fluorescence activated cell sorting procedures. While a variety of calcium channel blockers are known in the art, the preferred calcium channel blocker of the instant invention is verapamil. It is commercially available from Sigma Chemical Corporation. The effective concentration of verapamil can be determined empirically using techniques known in the art.

As alluded to above, the concentrations of the fluorescent dyes in the labeling solution must be non-toxic to the cells, yet high enough for the cells to take up an amount that can be detected in hybrids using the fluorescent activated cell sorter after the cells are fused and hybrids formed. For most dyes the concentration employed will be in the µg/ml range, and preferably in the range of 0.1 - 20 µg/ml. Hydroethidine and rhodamine 123 are preferably used at concentrations of about 0.1 10 µg/ml and 1 - 15 µg/ml, respectively. The most preferred concentrations of these dyes are 0.25 - 0.5 µg/ml, and 5 - 10 µg/ml for rhodamine 123 and hydroethidine respectively.

Other details regarding labeling of cells with suitable fluorescent dyes are the optimal time that the cells should be incubated with the dyes, as well as the incubation temperature. As mentioned above, the labeling period can vary considerably depending on the cell types used, as well as the concentration of the fluorescent dyes. However, the preferred incubation period is about 10 - 50 minutes, more preferred is 15 - 30 minutes, most preferred is 20 minutes. The cells can be incubated at a variety of temperatures, which will in turn affect the incubation period. The optimal time for the various parameters can be determined empirically. However, the preferred incubation temperature for cells incubated 20 minutes is about 37°C, for rhodamine 123 and 20 minutes at room temperature for hydroethidine. After the cell lines are labeled with the appropriate fluorescent dye, they are washed to remove residual dye and incubated in a physiological solution, preferably cell culture media, in preparation for carrying out the cell fusion procedures described below.

### Cell Fusion

The cell lines containing the appropriate fluorescent dyes are combined in a physiologically acceptable solution, and fused using standard cell fusion materials and methods. It is important to note that regardless of the procedure used, in those instances where the cell lines employed exhibit a significant rate of efflux of, for example, rhodamine 123 from rodent cells, that the fusing mixture will contain a suitable calcium channel blocker, preferably verapamil. Fusion can be effected using a variety of fusogens, the preferred fusogen, however, is polyethylene glycol. More preferred are polyethylene glycols having molecular weights in the range of 1500 to 4000. The two cell lines can be combined at different concentrations, however, it is preferred that about 10⁶⁻10⁷ cells of each cell line be utilized.

More specifically, the fusion procedure consists of combining about 10⁶-10⁷ cells of each cell line in a suitable cell culture media, with or without verapamil depending on the type of cell lines fused. The cell mixture is centrifuged to pack the cells, and the cells fused using polyethyleneglycol 1500. The technique employed is described by Kohler and Milstein, Nature, 1975, 256:495. Briefly, one procedure whereby cell hybridization can be achieved is by the addition of 0.5 ml of a 50% (v/v) solution of polyethyleneglycol 1500 by dropwise addition over 30 seconds at room temperature, followed by a 30 second incubation at 37°C. The cell suspension is slowly mixed with the addition of 20 ml of cell culture media containing 10% fetal calf serum. Next, cells are gently resuspended in cell culture medium containing 10% fetal calf serum and incubated for 2 -4 hours at 37°C prior to sorting the cells using a fluorescent activated cell sorter machine equipped with an argon laser that emits light at 488 or 514 mm. These wavelengths are particularly useful when using the dye pair hydroethidine and rhodamine 123. Hydroethidine intracellularly is converted to ethidium when excited at either 488 or 514 mm emits a red fluorescence. In contrast, rhodamine 123 when excited at either wavelength emits a yellow fluorescence. Thus, hybrid cells are expected to emit both red and yellow fluorescence.

### Cell Sorting

Hybrids present in the mixture of fused cells can be isolated using a fluorescent activated cell sorter machine. While a variety of such machines are available, we have found that a Coulter EPICS V, or a FACS III Cell Sorter produced by Beckon-Dickinson, Sunnyvale, California, perform adequately. Both are equipped with argon ion lasers. The laser is preferable used at a power setting of about 150 mW, and as mentioned above, at an excitation wavelength of 488, or 514 mm. Standard mirrors and filters are employed to be compatible with, and to collect the fluorescence emitted by, a select pair of fluorescent dyes. Similarly, standard techniques are used to set the cell sort windows that are used to select the hybrid cells, and are described by L. Karawajew, B. Micheel, O. Behrsing and M. Gaestel, J. Immunol. Methods, 1987, 96:265-270.

### Identification of Antibody Secreting Hybridomas

As mentioned above, the instant technique is particularly useful to isolate antibody secreting hybrid cells, preferably hybridomas, triomas, and hybrid hybridomas, or as they are more popularly known, quadromas. However, because not all hybrid cells isolated by fluorescence activated cell sorting will secrete antibody, those that do must be identified. As applied to detecting hybridomas, the most general approach involves assaying medium for antibody secreted by hybrid cells using a solid phase assay, although other techniques are known can be used. Similar techniques are available to detect membrane, or non-soluble antigens. In both instances, antigen is bound to a solid support, the support treated with a suitable blocking agent, and antigen detected either directly with labeled hybridoma antibody, or indirectly with a labeled molecule that binds to hybridoma antibody. The latter can be antibody, protein A, or other suitable binding agents. Such assays are known in the art, and are shown by Langone, J. and Van Vinakis, H., Methods of Enzymology, 92. Part E (1983).

Antibody secreted by triomas or quadromas can be identified by the methods described above, but additional assays may be performed to ascertain the bifunctional characteristics and properties of the antibody. As discussed above, antibody produced by triomas or quadromas is bispecific, that is, bifunctional in the sense of having binding affinities for two different antigens within a single antibody molecule. Antigen binding may be simultaneous or sequential. In order to confirm that triomas or quadromas can be isolated by the instant techniques antibody secreted by these cell lines can be characterized by any functional test which depends upon the binding of two different antigens by the same antibody molecule.

For example, bispecific antibody can be identified using bifunctional antigen sscreening assays to establish that the antibody does indeed recognize two distinct antigens, and thus is composed of two different antigen combining sites. Such assays are also known in the art, several of which are described in U.S. Patent Nos. 4,634,664; 4,714,681; and 4,474,893. These patents are hereby incorporated by reference. It is worth noting that U.S. Patent No. 4,634,664 describes triomas, whereas U.S. Patent Nos. 4,714,681 and 4,474,893 presents methods for making and identifying quadromas.

Cellular cytotoxicity assays can also be employed to identify antibody secreted by triomas or quadromas. Cellular cytotoxicity is thought to rely on cell surface receptors on cytotoxic cells, such as monocytes, macrophages, natural killers etc. These receptors are thought to be specific for, and to interact with membrane components on a target cell, thereby causing cell lysis by forming conjugates between the cytotoxic cell and the target cell. If the cytotoxic cell is positioned up against the target cell, cytotoxicity is enhanced. Bifunctional antibody can promote this process by binding to a target cell through one of its combining sites, and to the lysis promoting receptor on the cytotoxic cell through the second combining site, thereby joining the two cell types and causing the cytotoxic cell to deliver a kill signal. The materials and methods for performing these assays are generally known to those who work in this field. Representative assays are described by Mishell and Shiigi, in "Selected Methods in Cellular Immunology", p.130, eds. C. Henry and R. Mishell, publisher W.H. Freeman and Co., San Francisco (1984), and later Herlyn D., Herlyn M., Steplewski, Z., and Koprowski H., "Monoclonal Anti-Human Tumor Antibodies of Six Isotypes in Cytotoxic Reactions with Human and Murine Effector Cells", Cellular immunol, 1985, 92:105-114. The former reference describes a ⁵¹Cr release assay, whereas the reference shows a³H release assay involving measuring the release of tritiated thymidine from lysed cells.

After triomas or quadromas are identified using functional assays as referred to above, the bispecific nature of the antibody can be confirmed by determining the composition of isolated antibody preparations. Bispecific antibody should be composed of light and heavy chains of both fusing partners. Antibody can be purified from culture media or body fluids, as the case may be, by conventional immunoglobulin purification procedures such as ammonium sulfate precipitation, gel electrophoresis, dialysis, chromatography, and ultrafiltration, if desired. Ion exchange, size exclusion, or hydrophobic chromatography can be employed, either alone or in combination. Light and heavy chain analysis can be carried out using gel electrophoretic techniques, as well as other techniques known in the art.

Having generally described the invention, examples illustrative of its application will now be presented However, it will be understood by those skilled in the art that the examples are not intended to be restrictive in any way of the invention.

### Example I

### Fluorescent Dye Cytotoxicity Assay

Prior to forming hybrid hybndomas or quadromas of the cell lines 3G8 and 520C9, the cytotoxic effect of rhodamine 123 and hydroethidine was determined 3G8 is a murine hybridoma that secretes monoclonal antibody that recognizes human Fc receptor III present on various cell types, including monocytes, natural killer cells, and macrophages. It is described by Unkeless et al., Annual Review of Immunology, 1988, 6:251. 520C9 is a murine monoclonal antibody that recognizes an antigen present on breast cancer cells. It is described in U.S. patent 4,753,894.

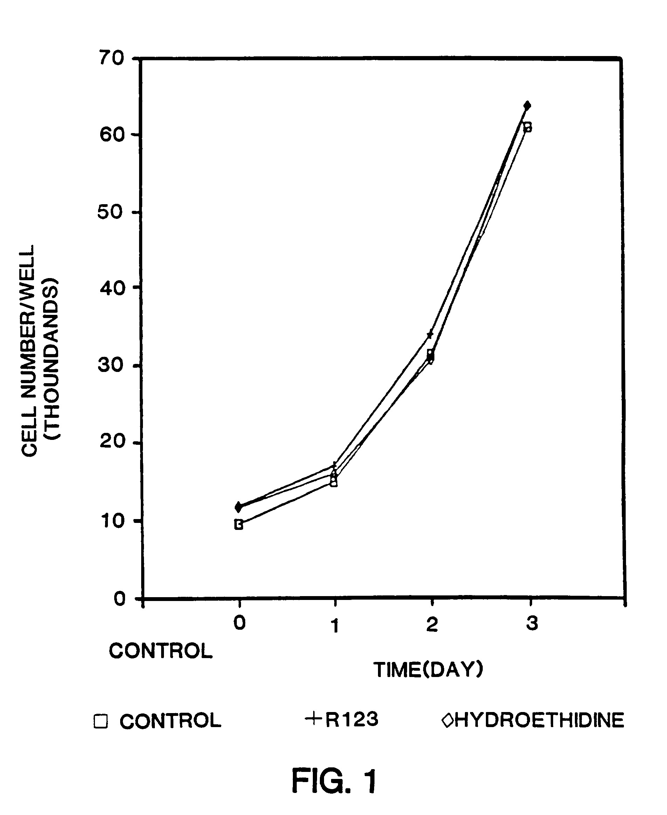

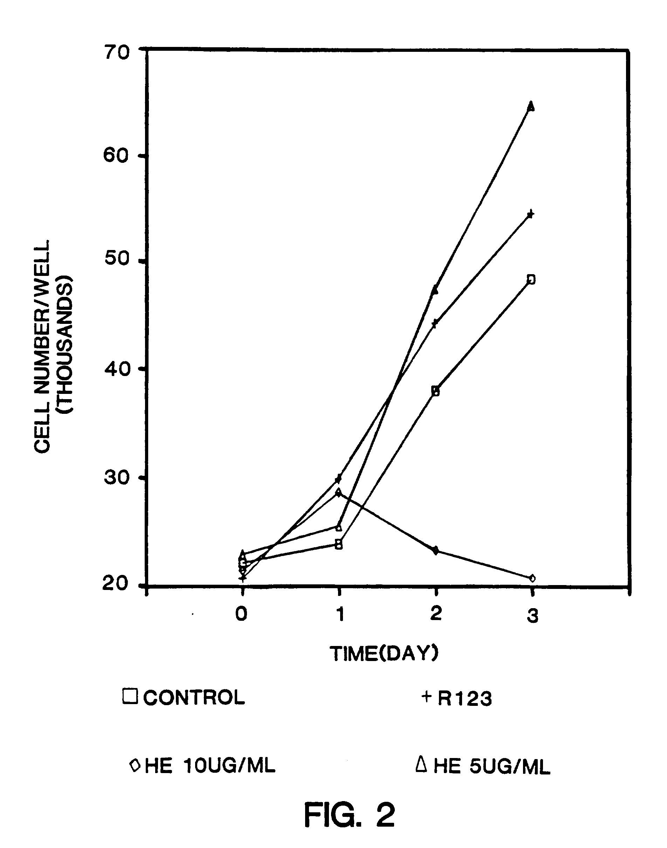

Cell growth and viability was determined using the MTT assay as described by Mosmann, T., above. In order to establish that the MTT assay was reflective of cell number, experiments were carried out to ensure that the assay exhibited lincarity over the appropriate range of cells. Each of the above cell lines was tested, and the results are shown in Figures 1, and 2 for 520C9 and 3G8 respectively. For both cell lines the assay was linear at least up to 10⁴ cells/well.

Next, the same cell lines were labeled in the presence or absence of either rhodamine 123 or hydroethidine, and subsequently cultured for three days during which cell number was measured. Cells were labeled in the presence of 0.5 µg/ml rhodamine 123 plus 10 µM verapamil, or 5 or 10 µg/ml hydroethidine. Between 10,000-20,000 cells/well were labeled with hydroethidine at room temperature, or rhodamine 123 at 37° C at room temperature in for 20 minutes, washed twice and then cultured in dye free medium. Figure 1 reveals that neither rhodamine 123 plus verapamil, nor hydroethidine (10 µg/ml) inhibit the growth of 520C9 cells. The growth rate and cell numbers are virtually identical when compared to cells grown in medium lacking this compounds.

Figure 2 shows the results for the cell line 3G8. Neither rhodamine 123 plus verapamil, nor hydroethidine at 5 µg/ml are inhibitory. However, hydroethidine at 10 µg/ml does inhibit 3G8 growth starting at about 24 hours.

### Example II

### Labeling of Cells with Fluorescent Dyes

The procedure for labeling cells with fluorescent dyes consisted of incubating about 2 x 10⁷ cells of 3G8, or 520C9 with either hydroethidine, 10 µg/ml, or rhodamine 123, 0.5 µg/ml. Cells incubated in rhodamine 123 also received 10 µM verapamil. Hydroethidine labeling occurred at room temperature and in the dark for 20 minutes. Rhodamine 123 labeling occurred at 37°C for 15 minutes, also in the dark. Following the incubation periods, the cell lines were washed twice with serumfree Iscove's medium containing 10 µM verapamil, and fused as described in Example III.

### Example III

### Formation/Isolation of Hybrid Hybridomas

Hybrid hybridomas were formed by fusing 3G8 cells to 520C9 cells. The initial step in the procedure consisted of labeling the cells as described in Example II, and subsequently fusing them with polyethylene glycol. The fusion procedure consisted of combining about 2 x 10⁷ cells of each cell line in 50 ml Iscove's medium , and pelleting the cells by centrifugation at 400 rpm for 5 minutes. The supernatant was removed, and the cells washed twice with serum free Iscove's medium containing 10 µM verapamil. This procedure was conducted at room temperature, while subsequent steps were done using solutions at about 37°C.

To the cell pellet consisting of 3G8 and 520C9 cells was added 180 µl of Iscove's medium without serum, and 100 ul of this mixture was transferred to a 50 ml centrifuge tube. This suspension was used to form the hybrids, while the remaining volume served as a control. Fusion was carried out by adding 0.4 ml of a 50% polyethylene glycol solution warmed to 37°C to the cell pellet over a 1 minute period. The mixture was gently swirled for an additional minute to expose as many cells as possible to the fusogen. Next, 0.5 ml of serum-free Iscove's medium was gently added to the mixture over a 1 minute period, and this step repeated once. 7 ml of serum-free Iscove's was added over a 2-3 minute period with continuous stirring, followed by transferring the mixture to a T-150 flask containing 80 ml of Iscove's medium supplemented with 10% fetal calf serum. The mixture was incubated at 37°C in an atmosphere of 95% air, 5% CO2 for 2-4 hours. Finally, the flask was removed from the incubator, and stood upright for 10 minutes to allow cells to settle to the bottom of the flask. 60 ml of the supernatant was aspirated off, and the ramaining mixture sorted to obtain hybrids using a FACS machine as described in Example IV, below.

### Example IV

Stained cell suspensions were analyzed and sorted using a Coulter EPICS V cell sorter. Rhodamine 123 and hydroethidine were excited at 488 nm with a argon laser at 150 mW power. A 488 nm dichroic mirror was used for collecting right angle light scatter, while a 550 nm dichroic mirror was used in combination with a 525 nm band pass filter and a 630 long pass filter to collect the rhodamine 123 and hydroethidine fluorescence, respectively. Forward angle light scatter (FALS), log right angle light scatter (LRALS), log green fluorescence and log red fluorescence were measured simultaneously. Dead cells and clumps were first gated out according to their FALS in combination with their LRALS properties. The second gate was constructed around the double-stained cells. Both gates were used to determine the percentage of double-stained cells (the second gate was gated on the first gate, i.e. FALS vs LRALS).

The double-fluorescent cells were sorted in either a round bottom 96-well microtiter plate, or a flat bottom 12-well plate under sterile conditions using a Coulter autoclone system. Single cells were sorted into 96-well plates, while large numbers of cells were sorted into a single well of a 12 well plate. The sorting gates were constructed on both windows (FALS vs LRALS, LGFL vs LRFL). Since both rhodamine 123 and hydroethidine stain only live cells, dead cells can be discriminated from the sort window. Similarly, clumps of live cells containing different fluorescence stained cells can be gated out according to their light scatter properties.

In order to ensure that hybrids would be soned using the above sorting parameters, the following control was done. 520C9 and 3G8 cells labeled with rhodamine and hydroethidine, respectively, were analyzed and the FACS profiles recorded.

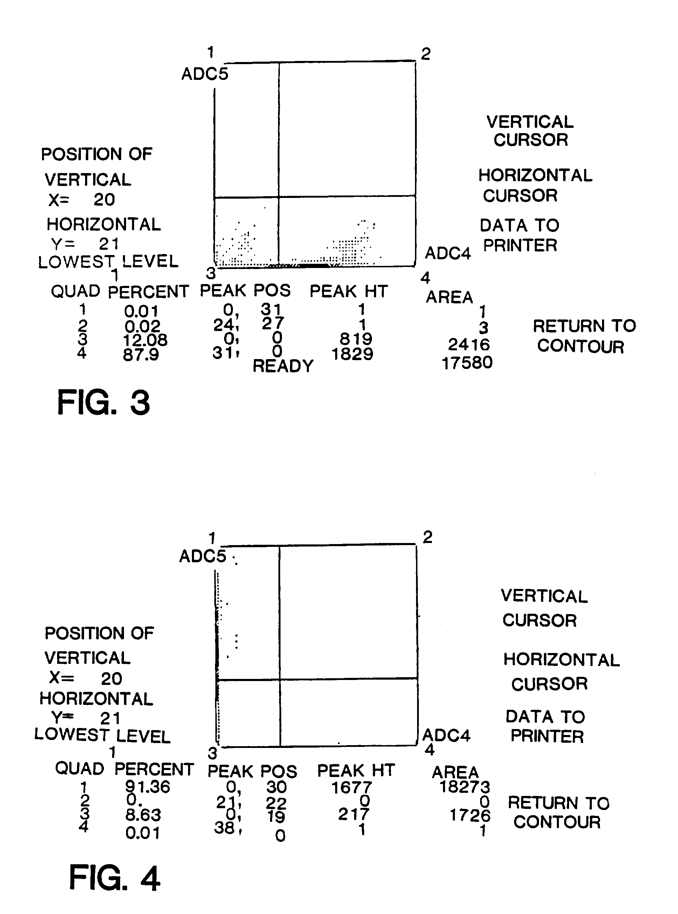

Figure 3 shows the dot-plot FACS profile of 520C9 cells labeled with rhodamine 123, while Figure 4 shows the profile of 3G8 cells labeled with hydroethidine. Few if any of either cell type fell into the cell son window set to select hybrid cells.

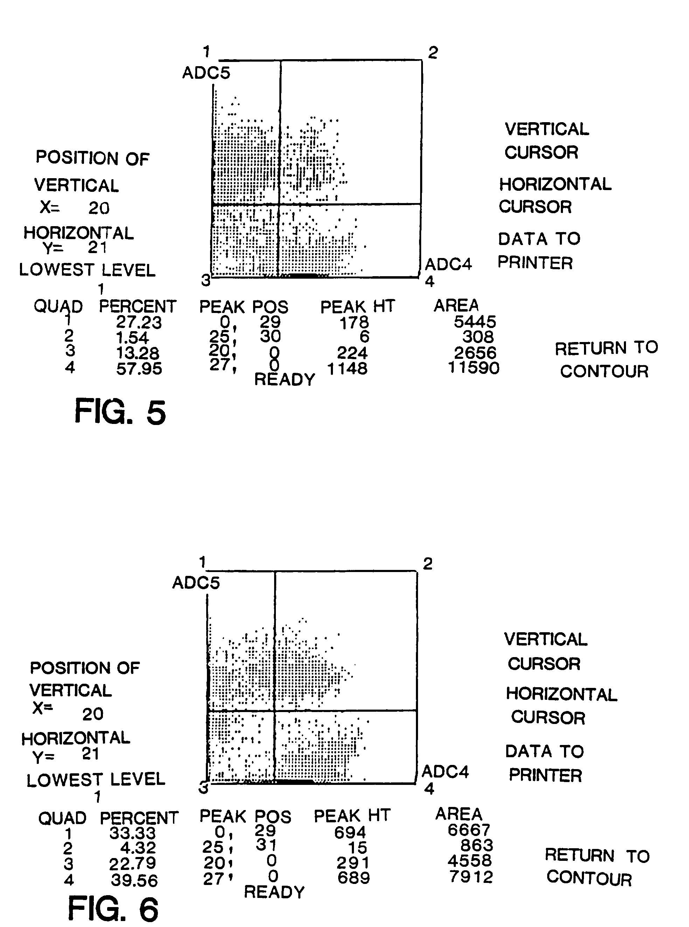

A second control was done to ensure that only hybnd cells would be sorted using the instant cell son parameters described above. A mixture of unfused 520C9 and 3G8 cells, that is, the control cells in Example III, was analyzed and the number of cells that fell in the hybrid cell window noted. Figure 5 shows that only about 1.54% of the cells present in the mixture fell within the sort window.

In contrast, when a mixture of fused 520C9 and 3G8 cells was sorted, there was a significant increase in the number of cells that fell within the hybrid cell son window settings. Figure 6 shows, that of the total cells present in the fusion mixture described in Example III, 4.32% fell within the hybrid cell window settings. This indicates that about 2% of the cells were fused.

### Example V

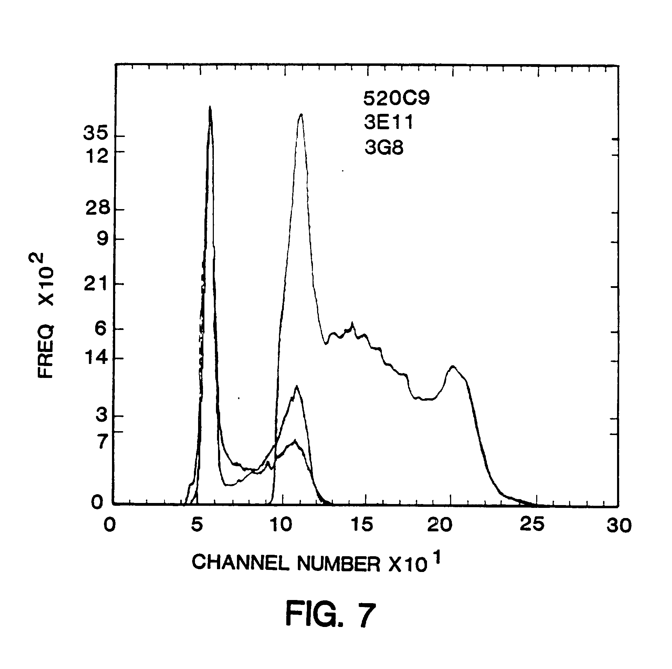

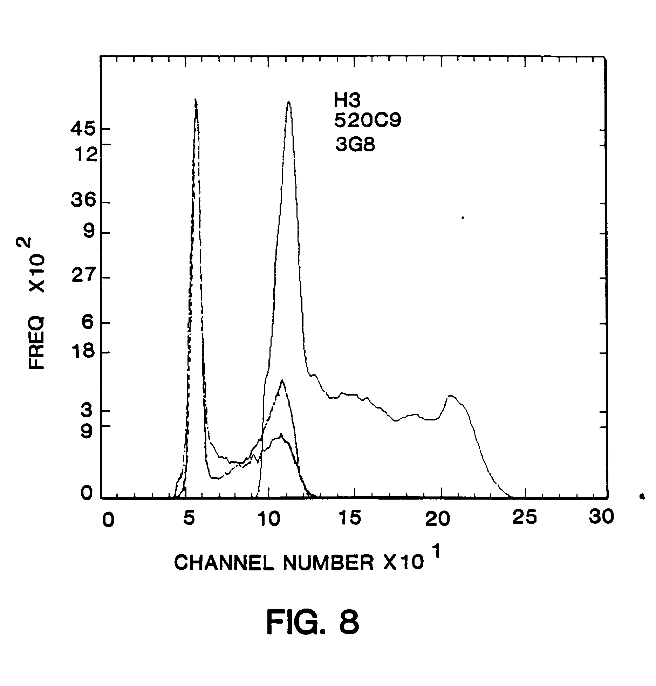

The DNA content of the parent cell lines,and hybrids derived therefrom, was determined in order to confirm that cells selected by the methods of Example IV were indeed hybrid cells resulting from the fusion of 3G8 to 520C9. Standard cell sorting techniques using the DNA stain chromomycin A3 were employed. The techniques are described by Young et al., Proc, Natl. Acad. Sci. USA, 1981, 78(12):7727. The cells were fixed in 70% methanol and stored at minus 20°C before being assayed. Two independent fusions were done, TS1 and TS3. TS1 produced six clones, while TS3 produced eight clones. Clones 3E11 and 4H3 were from TS1 and TS3, respectively. Figures 7 and 8, respectively, show that these quadromas exhibit about twice the DNA of the parent cell lines.

Taken together, these data establish that the procedures of the instant invention realize hybrid cells.

### Example VI

### Identification of Antibody Secreting Hybrid Hybridomas

Using the procedures described in the preceding examples, hybridomas 5A5 and 4H3, were generated from two independent 3G8 and 520C9 fusions, 5A5 from TS1 and 4H3 from TS3. These hybridomas were shown to secrete bispecific antibody by assaying for bifunctional biological activity initially using a chromium release cytotoxicity assay, which was subsequently confirmed using a thymidine release assay. These experiments were done using 5A5 or 4H3 culture medium containing antibody. Subsequently, 5A5 and 4H3 were subcloned, and the medium from several subclones also tested using the thymidine release assay. The subclones were 2B1 and 2D3 from 5A5; and 3D7 and 3E7 from 4H3. Further, antibody from one of the subclones, 2B1, was purified and tested to demonstrate bispecific activin. A control was also run consisting of medium from a hybridoma 3B5, obtained from the TS1 fusion, that does not secrete bispecific antibody. In addition, the subunit composition of the 2B1 bispecific antibody was analyzed to determine if it exhibited light and heavy chains derived from both 3G8 and 520C9 antibody.

Chromium labeled SKBr3 cytotoxicity assay: This assay is generally described by Mishell and Shiigi, in "Selected Methods in Cellular Immunonology". The cytotoxic cells were prepared as follows. 10 ml of heparinized blood was dispensed into a 50 ml tube and 30 ml of Hank's balanced salt solution containing calcium and magnesium (HBBS Ca-Mg) was added. The tube was inverted several times to mix the solution, and 10 ml of Ficoll-Hypaque slowly pipetted directly to the bottom of the tube. The tube was centrifuged for 25 minutes at 1250 rpm in a IEC DPR-6000 centrifuge at room temperature with the brake off, after which the tube was carefully removed to avoid mixing the solution. Next, the upper layer was removed using a Pasteur pipet down to just above mononuclear layer, which could be distinguished by its cloudy yellow-white appearance. The mononuclear cell layer was collected with a 10 ml pipet, with care not to disturb the red blood cell pellet. This procedure was used to process 30 mls of blood. The mononuclear cells so isolated were suspended in a volume of 50 ml with HBSS-Ca-Mg, and centrifuged for 20 minutes at 1250 rpm with the brake in the off position. The supernatant was aspirated to just above the cell pellet, and the cell pellet resuspended. The volume was increased to 50 ml with growth medium, an aliquot removed to determine cell number, and the cells repelleted at 1250 rpm for 10 minutes, again with the brake in the off position. The supernatant was aspirated off, the pellet resuspended and an appropriate amount of growth medium added to adjust the effector/target ratio, or E/T ratio, to 20/1 in a total volume of 0.2 ml/well. This amounted to 2 x 10⁶ effector cells/ml.

After the effector cells have been isolated and prepared as described above, or simultaneously therewith, the target cells, SKBr3, are prepared. The cells should be labeled with chromium while the cytotoxic cells are being prepared to ensure a highly viable cell population. The chromium labeling procedure that was followed is generally described by Mishell and Shiigi, above, with the following changes. SKBR-3 cells were grown to confluence in standard laboratory T150 cm flasks, and fed with fresh medium the day before the experiment. The cell monolayer was washed twice with 10 ml of HBSS-Ca-Mg, and the cells removed from the flask by washing the monolayer with 10ml of versene (EDTA), incubating the flask at 37°C/5%C02 for 3-5 minutes, and dislodging the cells by tapping the flask against a solid surface. Next, 10 ml of growth medium was added to resuspend the cells, and the mixture transferred to a 50 ml siliconized polypropylene tube. The volume was adjusted to 50 ml with growth medium, and an aliquot removed to determine the cell number. The cells were pelleted by centrifugation for 10 minutes at 1000 rpm, resuspended in 1 ml of growth medium, and 122 uCi of chromium-51 added per confluent flask of SKBr3 cells. The cell suspension was incubated for 45 minutes at 37°C with occasional agitation, after which 14 ml of HBSS-Ca-Mg was added, the cells pelleted, and the supernatant removed. The cells were washed once with 50 ml of HBSS-Ca-Mg and then once in growth medium to remove residual chromium, and resuspended in growth medium at a density of 2 x 10⁴ cells/0.1 ml. To standard laboratory 96-well culture plates, 0.1 ml of the labeled cells was added, plus 0.1 ml of medium alone or containing the appropriate reagent to be tested, that is, quadroma supernatant, heteroconjugate, or antibody secreted by the parent cell lines. The mixture was incubated at 37°C in an atmosphere of 95% air 5% CO₂ for 45 minutes to permit antibody to bind to the effector cells, followed by washing the cells to remove unbound antibody. Next, the supernatant was removed, 0.2 ml of effector cells/well added, and the effector/target cell mixture incubated for 3 hours at 37°C in an atmosphere of 95% air/5%CO₂. The cell mixture was pelleted, and 0.1 ml of supernatant removed and counted in a gamma counter to determine the per cent lysis and degree of cytotoxicity.

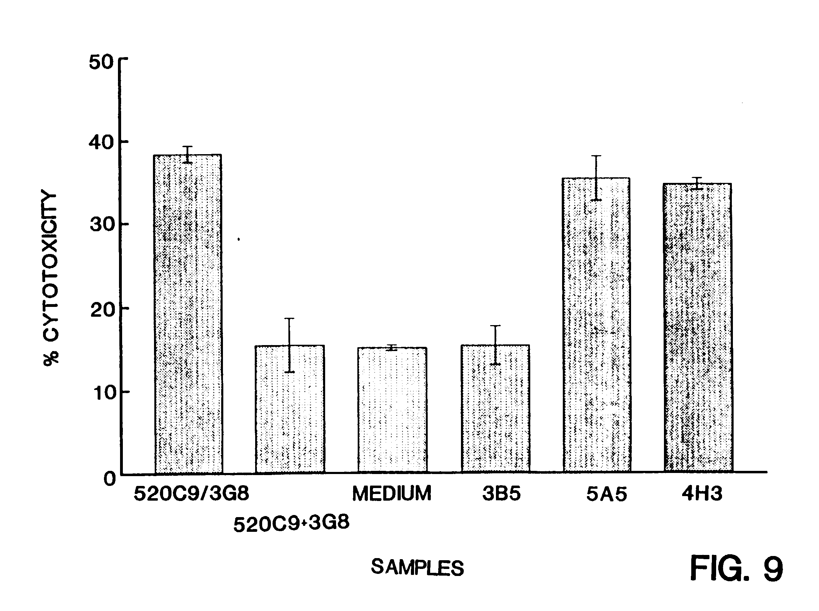

Figure 9 shows that the percent killing associated with antibody secreted by clones 4H3 and 5A5 is significantly greater than that attributed to antibody secreted by either parent cell line, 520C9 and 3G8, that were used to do the fusions that yielded 4H3 or 5A5. Indeed, there is nearly a two fold difference in killing caused by 4H3 and 5A5. The heteroconjugate, 520C9/3G8, showed elevated cytotoxicity similar to that observed for 4H3 and 5A5. Figure 9 also shows that control medium from 3B5 exhibits cytotoxic activity similar to that observed for the medium control. Thus, it is apparent that the quadromas, 4H3 and 5A5, and the subclones derived therefrom, produce antibody that has bispecific activity.

Tritium labeled SKBr3 cytotoxicity assay: Antibody produced by clones 5A5 and 4H3 and their corresponding subclones 2B1, 2D3, and 3D7, 3E7, respectively was assayed for bifunctional cellular cytotoxicity activity as follows. When considering the results, it is important to keep in mind that the antibodies were expected to exhibit binding to Fc receptor cells, as well as to breast cells that display the 520C9 antigen. Human buffy coat cells were pretreated with interleukin-2 and incubated with tritium labeled SKBr3 breast cancer cells in the presence of medium containing antibody, or under various control conditions. SKBr3 cells were labeled for 24 hours by growing the cells in medium supplemented with tritium labeled thymidine (2.5 uCi/ml). After the culture period, the cells were dislodged by trypsin and washed to remove free tritium. The experiment was run in serum free medium, and replicated using buffy coat cells from several human volunteers. Because the antibodies have combining sites that recognize an antigen on the buffy coat cells, as well as on SKBr3 cells, the antibodies should effect enhanced killing compared to their monospecific counterparts. Thus, one control consisted of determining the level of killing due to 520C9 and 3G8 antibodies, alone or in combination, relative to antibody produced by the hybrids. A second control consisted of comparing the level of killing due to a heteroantibody conjugate composed of 520C9 and 3G8. The latter was formed using chemical crosslinkers as described by Karpovsky, et al., J. of Experimental Medicine, 1984, 160:1686-1701. Finally, a medium control was also run.

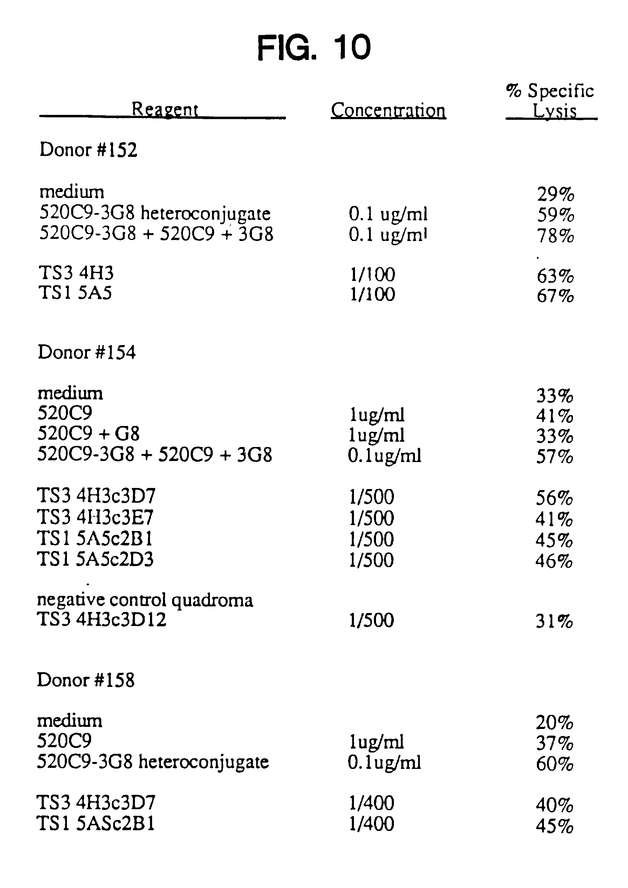

Figure 10 shows that antibody produced by either of the hybrids, 5A5 or 4H3, or their subclones, is more effective at killing SKBr3 cells than 520C9 or 3G8 antibody. However, the chemically linked heteroconjugate of 520C9 plus 3G8 is nearly as effective as antibody produced by the clones. Taken together,the results establish that bispecific antibody is produced by 5A5 and 4H3, and their subclones.

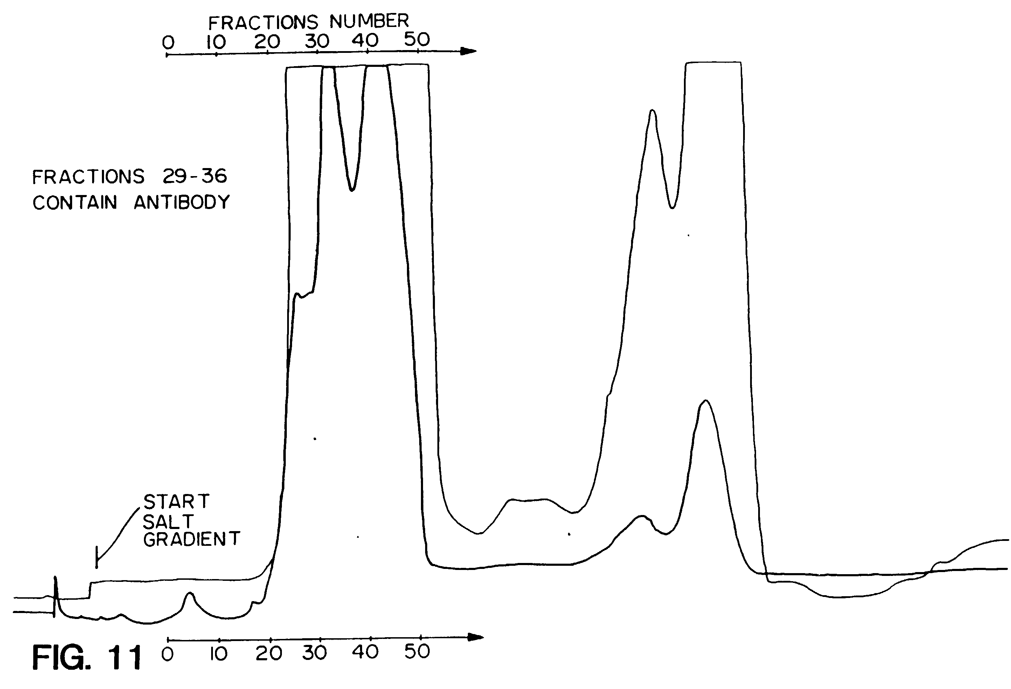

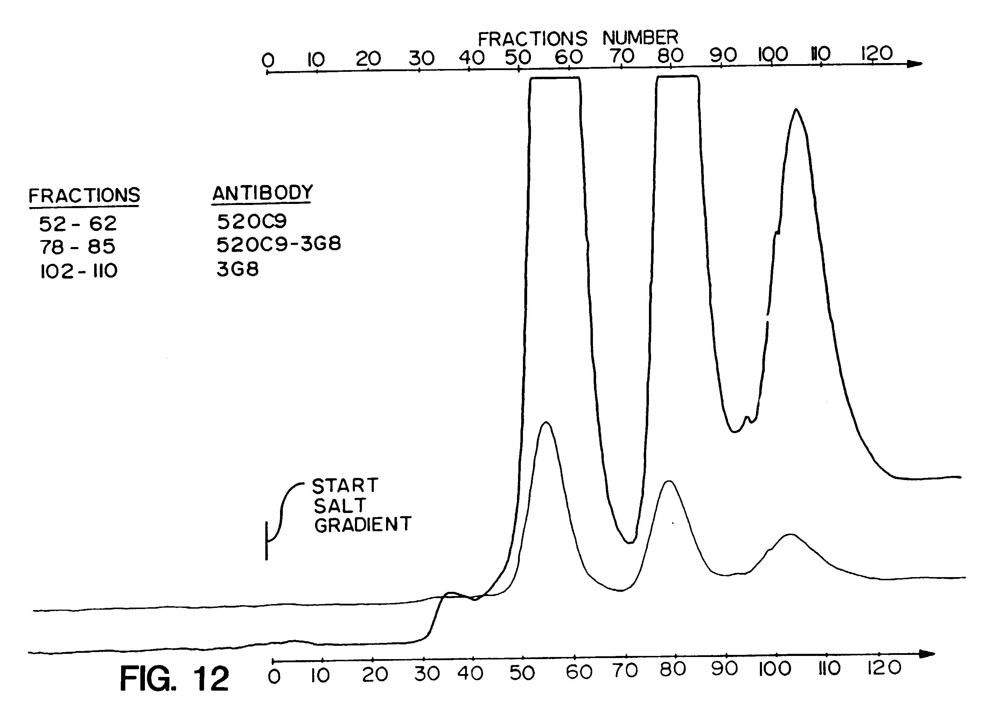

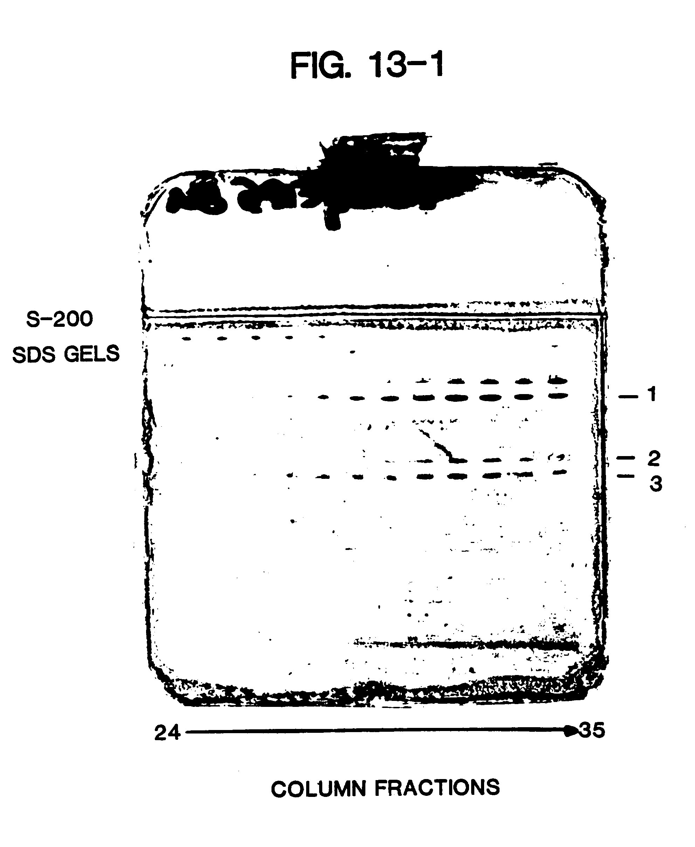

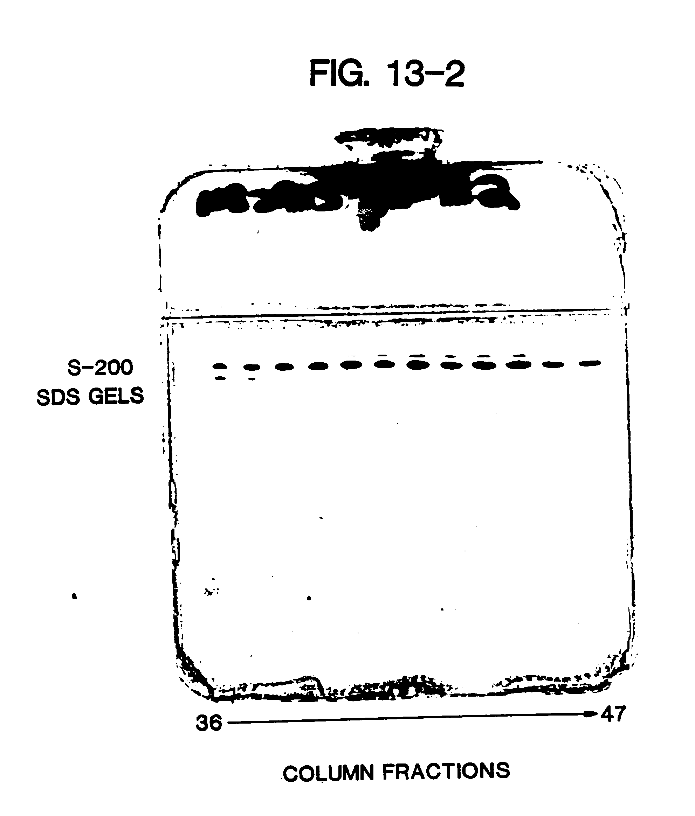

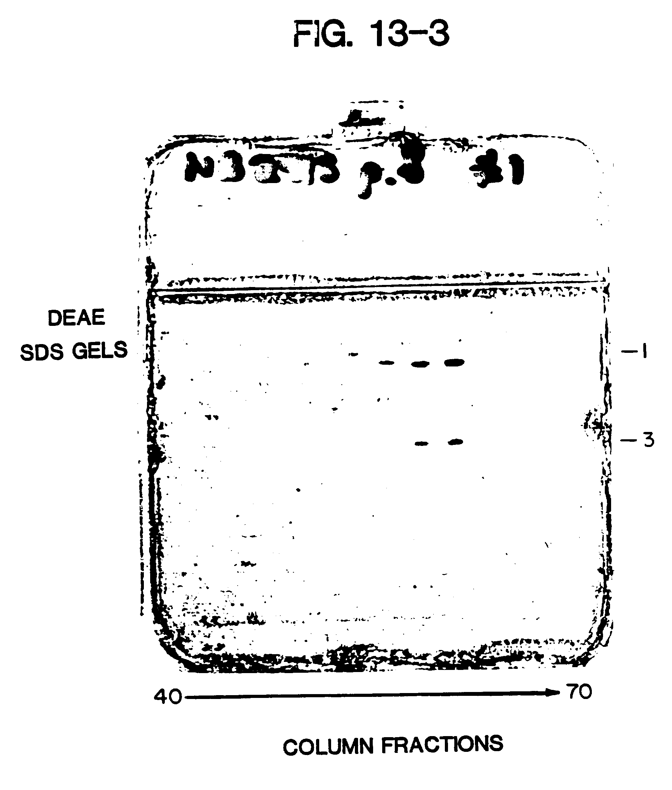

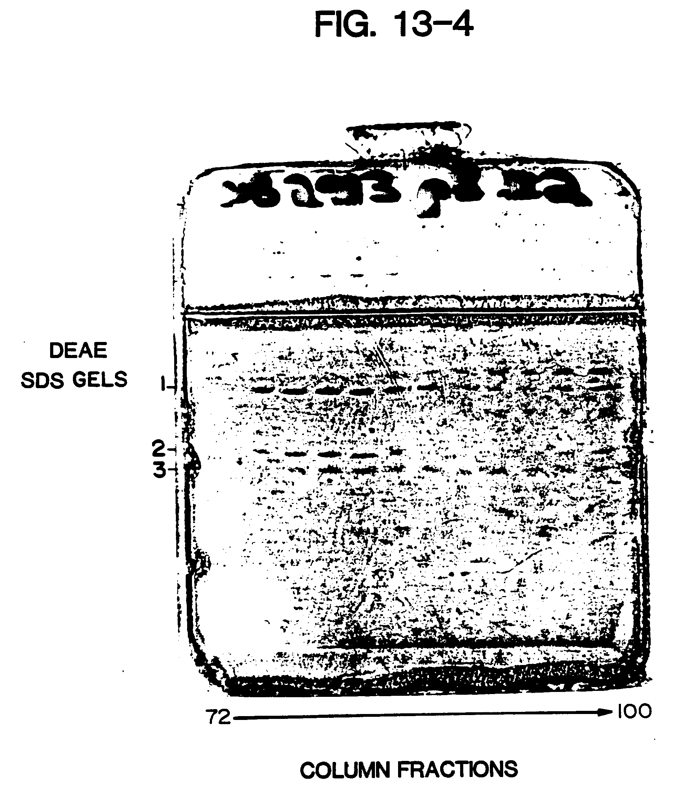

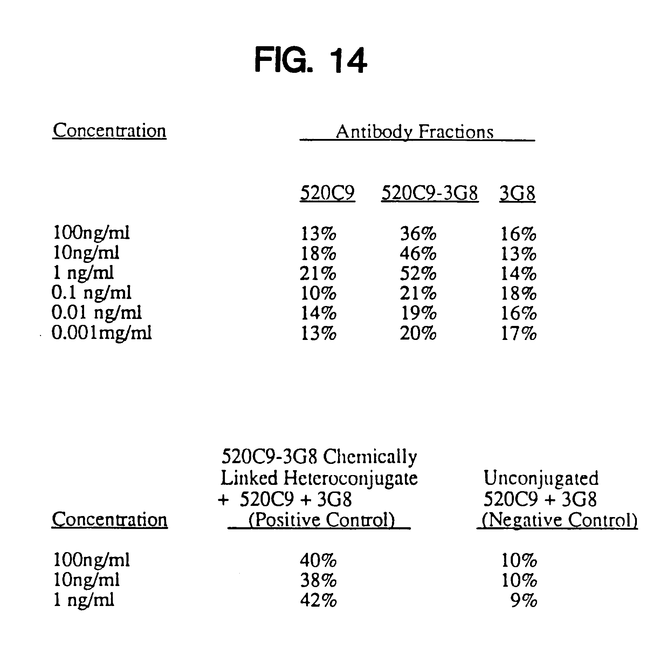

Antibody Characterization: Antibody from the 5A5 subclone, 2B1, was purified, characterized, and tested for cytotoxic activity as follows. 14.5 ml of defatted ascites fluid was chromatographed on a Sephacryl®-200 superfine column (Pharmacia Co.). The column, 26 mm in diameter and 850 mm long, was preequilibrated with 100 mM Tris-HCl buffer, pH 8.6. The same solution was used to elute the material at a flow rate of 1ml/min. The eluate was monitored at O.D.₂80, and 4ml fractions collected. Figure 11 shows the elution profile. Based on SDS PAGE gel electrophoresis across the column using 20% gels, fractions 29-36 contained antibody. These fractions were pooled, diluted four fold with water, and chromatographed on a DEAE-Sepharose® column (26mm x 130mm) previously equilibrated with 25mM Tris-HCl, pH 8.6. The column was rinsed once with 50ml of 25 mM Na phosphate, pH7.0, and antibody eluted with a 500 ml gradient of Na phosphate, 25 to 200 mM, pH 7.0. The eluate was monitored at O.D.₂80, and 3ml fractions were collected. The elution profile is shown in Figure 12. The fractions were analyzed for antibody using SDS PAGE gel electrophoresis using 20% gels, and fractions 52-62 pooled, as were fractions 78-85 and 102-110, and shown to contain 520C9 antibody, bispecific antibody, and 3G8 antibody, respectively. The gel profiles are shown in Figure 13. The three fractions were sterile filtered, and tested for cytotoxic activity against chromium labeled SKBr3 cells as described above. The results are shown in Figure 14. It is apparent from the Figure that the 2B1 bispecific pool, at antibody concentrations ranging from 100mg/ml to 1ng/ml, displays a marked increase in killing compared to the 520C9 or 3G8 pools.

## Claims

1. A method of producing a hybridoma capable of secreting antibody having bifunctional cellular cytotoxicity and recognising human Fc receptor III and the antigen recognised by the antibody produced by the 520C9 hybridoma comprising:
(a) contacting one of 3G8 cells, as described by Fleit et al, Proc. Natl. Acad. Sci. USA, 1982, 79: 3275-3279, and 520C9 cells, as described in US Patent No 4753894, with a physiological solution containing a first fluorescent dye for a time and at an effective concentration that permits said cells to become labelled with an amount of said first fluorescent dye detectable by a fluorescent activated cell sorting machine, said first fluorescent dye having a negligible rate of efflux from said cells;
(b) contacting the other of said 3G8 and 520C9 cells with a physiological solution containing a second fluorescent dye for a time and at an effective concentration that permits said other cells to take up an amount of said second fluorescent dye detectable by a fluorescent activated cell sorting machine and an effective amount of a calcium channel blocker that maintains said fluorescent dye at a detectible level inside said other cells;
(c) isolating said 3G8 and 520C9 cells from the respective physiological solutions;
(d) combining in a physiological solution the resulting 3G8 and 520C9 cells in the presence of a fusogen to produce a mixture containing hybrid and non-hybrid cells; and
(e) isolating hybrid cells from said mixture comprising passing said mixture through a fluorescent activated cell sorting machine.

2. A method of claim 1, wherein said fluorescent dyes are selected from rhodamine 123 and hydroethidine.

3. A method of claim 2, wherein hydroethidine is present in the respective physiological solution at a concentration of about 1-20 mg/ml and/or rhodamine 123 is present in the respective physiological solution at a concentration of about 0.5-1.0 mg/ml.

4. A method of any one of claims 1 to 3, wherein said calcium channel blocker is verapamil.

5. A method of claim 4, wherein verapamil is employed at a concentration of about 10 µM.

6. A hybridoma capable of secreting antibody having bifunctional cellular cytotoxicity and recognising human Fc receptor III and the antigen recognised by the antibody produced by the hybridoma 520C9 and obtainable by a method of any one of claims 1 to 5.

7. An antibody having bifunctional cellular cytotoxicity and recognising human Fc receptor III and the antigen recognised by the antibody produced by the 520C9 hybridoma.

## Patentansprüche

1. Verfahren zur Herstellung einer Hybridoma, die in der Lage ist, Antikörper mit bifunktioneller zellulärer Zytotoxizität zu sezernieren und menschlichen Fc Rezeptor III und das Antigen erkennt, das vom Antikörper erkannt wird, der von der 520C9 Hybridoma erzeugt wird, umfassend
(a) Kontaktieren einer der 3G8 Zellen, beschrieben von Fleit et al., Proc. Natl. Acad. Sci, USA, 1982, 79, 3275 - 3279, und von 520C9 Zellen, beschrieben in der US-Patentschrift 4 753 894, mit einer physiologischen Lösung, die einen ersten fluoreszierenden Farbstoff enthält, für eine solche Zeit und bei einer wirksamen Konzentration, die gestattet, daß die Zellen mit einer Menge an erstem fluoreszierenden Farbstoff markiert werden, der von einer fluoreszenzaktivierten Zellsortiermaschine erkennbar ist, wobei der erste fluoreszierende Farbstoff eine vernachlässigbare Effluxrate aus den Zellen hat;
(b) Kontaktieren der anderen der genannten 3G8 und 520C9 Zellen mit einer physiologischen Lösung, die einen zweiten fluoreszierenden Farbstoff enthält, für eine solche Zeitdauer und bei einer wirksamen Konzentration, die gestattet, daß die anderen Zellen eine Menge des zweiten fluoreszierenden Farbstoffs aufnehmen, der von einer fluoreszenzaktivierten Zellsortiermaschine erkannt werden kann, und eine wirksame Menge eines Calciumkanalblockers, der den fluoreszierenden Farbstoff auf einem erkennbaren Niveau innerhalb der anderen Zellen hält;
(c) Isolieren der 3G8 und 520C9 Zellen aus den entsprechenden physiologischen Lösungen;
(d) Vereinigen der erhaltenen 3G8 und 520C9 Zellen in einer physiologischen Lösung in Gegenwart eines Fusogens zur Bildung eines Gemisches, das Hybrid- und Nicht-Hybridzellen enthält; und
(e) Isolieren der Hybridzellen aus dem Gemisch durch Hindurchleiten des Gemisches durch eine fluoreszenzaktivierten Zellsortiermaschine.

2. Verfahren nach Anspruch 1, wobei die genannten fluoreszierenden Farbstoffe ausgewählt sind aus Rodamin 123 und Hydroethidin.

3. Verfahren nach Anspruch 2, wobei das Hydroethidin in der entsprechenden physiologischen Lösung in einer Konzentration von etwa 1 bis 20 mg/ml und/oder das Rodamin 123 in der entsprechenden physiologischen Lösung in einer Konzentration von etwa 0,5 bis 1,0 mg/ml vorliegt.

4. Verfahren nach einem der Ansprüche 1 bis 3, wobei der Calciumkanalblocker Verapamil ist.

5. Verfahren nach Anspruch 4, wobei das Verapamil in einer Konzentration von etwa 10 µM verwendet wird.

6. Eine Hybridoma, die in der Lage ist, Antikörper mit bifunktioneller zellulärer Zytotoxizität zu sezernieren, der menschlichen Fc Rezeptor III und das Antigen erkennt, das vom Antikörper erkannt wird, das von der Hybridoma 520C9 erzeugt wird, erhältlich nach einem Verfahren nach einem der Ansprüche 1 bis 5.

7. Antikörper mit bifunktioneller zellulärer Zytotoxizität, der menschlichen Fc Rezeptor III und das Antigen erkennt, das vom Antikörper erkannt wird, der vom 520C9 Hybridoma erzeugten Antikörper erkannt wird.

## Revendications

1. Méthode de production d'un hybridome capable de secréter un anticorps ayant une cytotoxicité cellulaire bifonctionelle et reconnaissant le récepteur humain III du Fc et l'antigène reconnu par l'anticorps produit par l'hybridome 520C9, comprenant :
(a) la mise en contact d'un des types de cellules 3G8, comme décrit par Fleit et al., Proc. Natl. Acad. Sci. USA, 1982, 79 : 3275-3279, et 520C9, comme décrit dans le brevet américain N° 4753894, avec une solution physiologique contenant un premier colorant fluorescent pendant un laps de temps et à une concentration efficace permettant que lesdites cellules soient marquées par une quantité dudit premier colorant fluorescent détectable par un appareil de triage de cellules activées par fluorescence, ledit premier colorant fluorescent ayant une vitesse d'écoulement hors des cellules négligeable ;
(b) la mise en contact de l'autre type desdites cellules 3G8 ou 52C09 avec une solution physiologique contenant un second colorant fluorescent pendant un laps de temps et à une concentration efficace permettant que lesdites autres cellules absorbent une quantité dudit second colorant fluorescent détectable par un appareil de triage de cellules activées par fluorescence et une quantité d'agent bloquant le canal calcique suffisante pour maintenir ledit colorant fluorescent à un niveau détectable dans lesdites autres cellules ;
(c) l'isolement desdites cellules 3G8 et 520C9 de leur solution physiologique respective ;
(d) la combinaison dans une solution physiologique des cellules 3G8 et 520C9 obtenues, en présence d'un agent de fusion, afin de produire un mélange contenant des cellules hybrides et non-hybrides ; et
(e) l'isolement des cellules hybrides dudit mélange, comprenant le passage dudit mélange à travers un appareil de triage des cellules activées par fluorescence.

2. Méthode selon la revendication 1, dans laquelle lesdits colorants fluorescents sont choisis parmi la rhodamine 123 et l'hydroéthidine.

3. Méthode selon la revendication 2, dans laquelle l'hydroéthidine est présente dans la solution physiologique respective à une concentration d'environ 1 à 20 mg/ml et/ou la rhodamine est présente dans la solution physiologique respective à une concentration d'environ 0,5 à 1,0 mg/ml.

4. Méthode selon l'une des revendications 1 à 3, dans laquelle ledit agent bloquant le canal calcique est le vérapamil.

5. Méthode selon la revendication 4, dans laquelle le vérapamil est employé à une concentration d'environ 10 µM.

6. Un hybridome capable de secréter un anticorps ayant une cytotoxicité cellulaire bifonctionnelle et reconnaissant le récepteur humain III du Fc et l'antigène reconnu par l'anticorps produit par l'hybridome 520C9 et que l'on peut obtenir par la méthode de l'une des revendications 1 à 5.

7. Un anticorps ayant une cytotoxicité cellulaire bifonctionnelle et reconnaissant le récepteur humain III du Fc et l'antigène reconnu par l'anticorps produit par l'hybridome 520C9.
